Europäisches Patentamt

(19) European Patent Office

Office européen des brevets

(11) Numéro de publication: **0 049 715**

**A1**

(12)
# DEMANDE DE BREVET EUROPEEN

(21) Numéro de dépôt: **80200934.0**

(22) Date de dépôt: **02.10.80**

(51) Int. Cl.³: **A 61 K 7/06**
**A 61 K 7/48**

(43) Date de publication de la demande:
**21.04.82 Bulletin 82/16**

(84) Etats contractants désignés:
**BE CH DE FR LI**

(71) Demandeur: **Mengoli, Franco**
**Viale Filopanti, 4**
**I-40126 Bologna 7(IT)**

(72) Inventeur: **Mengoli, Franco**
**Viale Filopanti, 4**
**I-40126 Bologna 7(IT)**

(74) Mandataire: **Sassatelli, Franco**
**c/o INIP Ufficio Internazionale Brevetti per Deposito di**
**Brevetti e Marchi via Mazzini, 170**
**I-40139 Bologna(IT)**

(54) **Aromatisation naturelle de produits pour l'hygiene personelle.**

(57) L'invention se réfère à une aromatisation naturelle de produits pour l'hygiène personnelle, qui prévoit l'emploi de formulation de base pour la production de solutions balancées à densité élevée, aptes à préserver la conservation naturelle de herbes et fleures officinales et aromatiques immersées en elles pour en absorber, dans un certain temps, les aromes avec solubilisations des principes actifs contenus dans les espèces botaniques.

EP 0 049 715 A1

- 1 -

Aromatisation naturelle de produits pour l'hygiene personelle

L'invention se réfère à l'execution de produits pour trai
tements épidermiques à aromatisation naturelle caractéri
sés substantiellement par l'englobement direct des principes actifs de herbes et fleures officibales et aromati
ques posées en immersion en conteneurs de solutions balan
cées ayant densité élévée afin de permettre une lente action d'extraction des aromes. Cela est consenti par le
fait que le balancement des formulation permet pour un
temps très long la permanence de la conservation naturel
le des dites herbes et fleures, alors que leurs aromes,
en un certain temps, sont complètement absorbés par les
solutions.

Dans les traitements épidermiques, des essences composées sont actuellement employées qu'on obtient par voie syn
thétique. Ces produits sont peu tolérés et peuvent causer
des dommages, tens que l'écaillage de la peau, des rougis
sements ou autre. Les essais effectués actuellement pour
absorber les aromes de fleures et plantes ayant une action spécifique, dans le traitement épidermique à effectuer, ont jusqu'ici trouvé un obstacle insurmontable dans
les types de formulations employées qui causaient la vi-

- 2 -

te périssement de la partie botanique en immersion.

Le trouvé permet la solution du problème en consentant le direct emploi des bien connues propriétés curatives et régénérantes de quelques espèces botaniques.

Une formulation prévoit la réalisation d'un produit spécifique pour le lavage des cheveux aux fleurs de camomille et inclut: Laurilétère sulphat naturel de coco, huile naturelle coco, protéine collagénique animale, extrait naturel de camomille, glycole propilénique et résine de polycondensation, sels inorganiques 2% - PH - 7,00 (neutre), matière active 29,8%, viscosité 6000-7000 cP. La combination des deux huiles naturelles introduites permet d'une côté la lente extraction aromatique vers les fleurs de camomille et, de l'autre, une pénétration vigoureuse et prononcée vers la peau et le bulbe du cheveu sec des extraits de camomille qui provequent une nouvelle épitè lisation et favorisent le dérougissement de la peau.

Le laurilétère naturel de coco présente: autre le pouvoir détergeant élevé, à l'indépendence écumegène de la dureté de l'eau et à la presque total absence d'irritabilité cutanée, une exportation aisée par rinçage de l'écume qui a été formée et donne aux cheveux brillantesse et bonne disposition à la coiffure; la amide de coco, outre à développer son pouvoir émollient sur la peau, stabilise la écume en la rendant plus fine et délicate, aussi bien que compacte et durable.

Le point de troublement du produit et au-dessous de 0°C.,

- 3 -

ce qui permet une stabilité dans le temps même à basses températures. L'index de toxicité DL$_{50}$ est en général au dessous de 2 gr./Kg. Le dérivé protéique collagénique animal favorise la clart) du cheveu et son douillet, con jointement à une nourriture protéinique élevée.

Pour les cheveux gras, une formulation est prévue aux ba ies de genièvre comprenant: laurilétère sulphat naturel de coco, hiules naturelles extractives, bétaine naturelle amidique de coco, protéine collagénique animale, extrait naturel de genièvre, additif acide naturel à viscosité 20 °C. = 6000 - 7000 cP; baies de genièvre à absence absolue de colorants; sels inorganiques totaux 2,2% - glicoles, résine de polycondensation - PH-6,5 (faiblement acide) et matière active = 21,4%. La bétaine naturelle employée en haute concentration consente une compatibilité cutanée totale au produit très élevée, innocuité vers les yeux et les parties délicates du visage, abaissement de la curve d'irritabilité dermatologique, fort effet as souplissant-protectif du cuir vhevelu. Les cheveux résul tent aptes à être coiffés, luisants et doués avec anti-staticité. L'écume est abondante, compacte, finement dé licate, ayant pouvoir dégraissant élevé grâce aussi à l'additif acide. L'extrait de genièvre et l'arome provenant des baies déterminent le rétablissement de la circulation cutanée et à la base des bulbes avec enlèvement des graisses présentes. A complément on prévoit l'emploi d'essence naturelle de citron sicilien obtenu par pressu rage des écorces. Le point de troublement résulte au-des sous de 0°C. L'index de toxicité DL$_{50}$ est extrèmement bas même par l'effet de la b)taine de coco qui est douée

- 4 -

d'un $DL_{50}^{-}$ propre = 1,85 gr./Kg. Cette formulation consente une haute inhibition de la flore bactérique.

Pour des cheveux normaux ou fragils, on prévoit une formulation protéique au romarin qui inclut: laurilétère sulphat naturel de coco, huiles naturelles extractives, bètaine et amides naturelles de coco; protéine collagénique animale; extrait naturel de romarin. La viscosité 20°C.= 6000 - 7000 cP; rameaux de romarin; sels inorganiques totaux 2,0% - glicols; PH-7 (neutre) - Matière active = 24,0%.

La formulation balancée emploie l'action combinée des deux dérivés amidiques bétainiques de coco qui sont présents séparément dans les deux formulations précédentes. En résulte une action )molliente, délicatement détergente, la écume fine et compacte, pouvoir bactéricide. La formule est completée par une huile ayant un pouvoir extractif à haute pénétration cutanée. Le romarin déploie une action renforçante et purifiante.

Pour l'exécution d'écume à bain, la formulation suivante de base a été prévue: laurilétère sulphat naturel de coco; huile naturelle extractive-glicols; alquibétaine de coco naturelle: amide glycérique naturelle de coco; sels inorganiques totaux au-dessous du 2,5%; PH = 7 (neutre) - Matières actives = 23,8%; Additifs: essences et extraits naturels contenu total 2,5 + 3,5; viscosité 20° C. = 6000 - 7000 cP - Index de toxicité $DL_{50}$ très bas; résine de polycondensation. Les amides déploient protection cutanée, inhibition à la flore bactérique, dérou-

- 5 -

gement et en même temps pouvoir émollient prononcé et stabilisant avec écume fine, délicate et compacte. La huile extractive, outre à déployer une action solubilisant aromatique, agit avec une forte pénétrabilit) et action réengraissante.

En version d'agrément, l'emploi est prévu dans les formulation pour écumes à bain des plantes suivantes: camomille romaine, romarin, menthe, sauge lavande, cèdre, bergamote, genièvre, rose et oeillet. Une ligne médicamenteuse est prévue qui inclut trois produits et une série de formulations basées sur composition de base: 1) Détergent curatif défurfirant protéinique; 2) Conditionant balsamique pour cheveux; 3) Bain de écume curatif anticelluliti que; 4) Huiles pour bain drmatologiques aux huiles essentielles naturelles.

La formulation du détergent curatif défurfirant protéinique inclut: le laurilétère sulphat naturel de coco; huile naturelle surgraissante; alcohols; graisses naturelles; bétaine naturelle de coco; protéine collagénique animale; sel complexe de zinc; extrait naturel d'ortie; essence naturelle de menthe; menthe pipérite en feuilles; sels inorganiques totaux = 2,7%; matière active = 22,8%; PH= 6,5 (faiblement acide) – Viscosité 20 °C. = 6000 – 7000 cP. Outre les premiers ingédients base naturels et le dérivé protéique animal, il y a aussi un sel complexe de zinc déployant une action défurfirant et inhibant bactérique.

Le conditionnant balsamique pour cheveux inclut en formu lation: polymères quaternaire; résine de condensation po lyglycolique aminique; alquilbétaine naturelle de coco; alcohols; graisses naturelles; essence à composants natu rels; sels inorganiques totaux - 0,2%; matière active - 8,4%; PH- 7 (neutre); Viscosité 20°C. - 4000 - 4500 cP. Ce baume est curatif pour cheveux et est basé sur l'emploi de polymères quaternaires. Ils donnent soieuseté aux che veux, en améliorant la possibilité de les coiffer et en réduisant la staticité. L'effet sul la peau est de la ren dre lisse et soieuse. Leur caractéristique d'être des po lymères détermine la propriété de maintenir la plie.

La formulation du bain d'écume curatif anticellulitique emploie: laurilétère sulphat de coco naturel; alchil-bé taine naturelle de coco; dérivé aminique naturel de coco; huile naturelle surgraissante; extrait de chêne marin; feuilles de hamamélis; sels inorganiques totaux - 2%; PH= 6,5 (faiblement acide); Viscosité 20° C. - 5000 - 6000 cP. En cette formulation outre les détergents naturels de coco et les émollients protectifs pour la peau à base de amides de coco, il y a aussi le chaîne marin et l'hamamélis.

La formulation des huiles à bain dermatologique, aux hui les essentielles naturelles, incluent: un composant huil leux dérivé de l'alcohol décilique; un composant huilleux dérivé de l'acide miristique; alquil-bétaine naturelle de coco; huile naturelle d'olive; huiles essentielles na turelles; extrait naturel de tilleul; composants huilleux totaux = 50%. Les deux composants huilleux en particulier consentent une élevée compatibilité cutanée, absence d'ir ritation, forte pénétrabilité de l'épiderme, haut pouvoir extractif et dissolvant envers les principes actifs et pouvoir surgraissant élevé. En pratique les dites formulations de base pourront être opportunément variées et intégrées avec d'autres composants dans les buts d'emploi et de possible utilisation des espèces botaniques en général.

Revendications:

1) Aromatisation naturelle de produits pour l'hygiène personelle, carctérisée par le fait qu'elle prévoit l'emploi de formulation de base pour la production de solutions balancées à densité élevée, aptes à préserver la conservation naturelle de herbes et fleures officinales et áromatiques immersées en elles pour en absorber, dans un certain temps, les aromes avec solubilisations des principes actifs contenus dans les espèces botaniques.

Le procédé permet particularités d'emploi pour des produits détergents et curatifs pour le lavage des cheveux à des traitements épidermiques en général. La formulation base prévoit l'emploi de: laurilétère sulphat naturel de coco; huiles et graisses naturelles; amide naturel de coco; protéine collagénique animale; glycole propilénique et dérivé quaternaire d'ammonium.

2) Aromatisation naturelle de produits pour l'hygiène personnelle, selon la revendication précédente, caractérisée par le fait qu'une formulation prévoit la production d'un spécifique pour le lavage de cheveux secs aux fleures de camomille et inclut: laurilétère sulphat naturel de coco, huiles naturelles extractives, amide émollient naturel de coco, protéine collagénique animale, extrait naturel de camomille, glycole propilénique et résine de policondensation - sels inorganiques 2% - PH = 7 (neutre)- Matière active 29,8% - Viscosité 6000÷7000 cP. La combination des deux huiles introduites permet, d'un côté, la lente extraction aromatique vers les fleurs de camomille et, de l'autre, une pénétration vigoureuse et marquée vers l'épiderme et le bulbe du cheveu sec des extraits de camomille qui provoquent une nouvelle épitélisation et favorisent le dérougement cutané. Le laurilétère naturel du coco présente, outre le pouvoir détergent élevé, l'indépendance écumogène de la dureté de l'eau et l'absence presque totale d'irritabilité cutanée, une aisée exportatione de l'écume formée par rinçage et donne aux cheveux brillantesse et prédisposition à la coiffure; l'amide de coco, outre son pouvoir émollient expliqué sur la peau, stabilise l'écume en la rendant plus fine et délicate aussi bien que compacte e durable. Le point de troublement du produit est au-dessous de 0° C., ce qui permet une stabilité dans le temps même à basses températures. L'index de toxicité DL$_{50}$ est en général au-dessous de 2 gr./Kg. Le dérivé collagénique animal favorise la brillantesse du cheveu et son douillet conjointement à la nourriture protéinique élevée.

3) Aromatisation naturelle de produits pour l'hygiène personnelle, selon les revendications précédentes, caractérisée par le fait que, pour les cheveux gras, une formulation est prévue aux baies de genièvre comprenant: laurilétère sulphat naturel de coco, huiles naturelles extractives, bétaine naturelle amidique de coco, protéine collagénique animale, extrait naturel de genièvre, additif acide naturel ayant viscosité 20° C. = 6000 ÷ 7000 cP; baies de genièvre avec absence absolue de colorants; sels inorganiques totaux 2,2% - Glycoles - Résine de polycondensation, PH = 6,5 (faiblement acide et matière active = 21,4%.

4) Aromatisation naturelle de produits pour l'hygiène personnelle, selon les revendications précédentes, caractérisée par le fait que, pour cheveux normaux ou fragiles, une formulation protéique est prévue au romarin incluant: laurilétère sulphat naturel de coco, huiles naturelles extractives, bétaine et amide naturelles de coco; protéine collagéniques animale; extrait naturel de romarin. La viscosité 20°C. = 6000 ÷ 7000 cP; rameau de romarin; sels inorganiques totaux 2,0% - glicoles; PH = 7 (neutre) - Matière active = 24,0%.

5) Aromatisation naturelle de produits pour l'hygiène personnelle, selon les revendication précédentes, carctérisée par le fait que, pour obtenir des écumes de bain, la formulation suivante est prévue comme base: laurilétère sulphat naturel de coco; huile naturelle extractive-glycoles; alquibétaine de coco naturelle; amide glycérique naturelle de coco; sels inorganiques totaux au-dessous du 2,5%; PH = 7 (neutre) - matière actives = 23,8%; additifs; essences et extraits naturels contenu total 2,5 ÷ 3,5; viscosité 20°C. = 6000 ÷ 7000 cP - Index de toxicité $DL_{50}$ très bas; résine de polycondensation.

6) Aromatisation naturelle de produits pour l'hygiène personnelle, selon les revendications précédentes, caractérisée par le fait que la formulation du détergent curatif inclut: le laurilétère sulphat naturel de coco; huile naturelle surgraissante; alcohols, graisses naturelles, bétaine naturelle de coco; protéine collagénique animale; sel complexe de zinc; extrait naturel de hortie; essence naturelle de menthe; menthe pipérite en feuilles; sels inorganiques totaux = 2,7%; matière active = 22,8%; PH =

- 11 -

6,5 (faiblement acide) - Viscosité 20°C.= 6000 ÷ 7000 cP.

7) Aromatisation naturelle de produit pour l'hygiène personnelle, selon les revendications précédentes, caractérisée par le fait que le conditionnant balsamique pour cheveux inclut en formulation: polymères quaternaires; résine de condensation polyglycolique aminique; alquilbétaine naturelle de coco; alcohols; graisses naturelles de coco; essence à composants naturels; sels inorganiques totaux = 0,2%; matière active = 8,4%; PH = 7 (neutre); viscosité 20°C.= 4000 ÷ 4500 cP. Ce baume est curatif pour cheveux et est basé sur l'emploi de polymères quaternaires.

8) Aromatisation naturelle de produits pour l'hygiène personnelle, selon les revendications précédentes, caractérisée par le fait que la formulation du bain d'écume curatif anticellulique emploie: laurilétère sulphat de coco naturel; alchil-bétaine naturelle de coco; dérivé amidique naturel de coco; huile naturelle surgraissante; extrait de chaine marin; feuilles de hamamélis; sels inorganiques totaux = 2%; PH = 6,5 (faiblement acide); viscosité 20°C. = 5000 ÷ 6000 cP.

**0049715**

Numéro de la demande

**RAPPORT DE RECHERCHE EUROPEENNE**

EP 80 20 0934

Office européen
des brevets

| Catégorie | Citation du document avec indication, en cas de besoin, des parties pertinentes | Revendica-tion concernée |
|---|---|---|
| | DE - A - 2 231 887 (H. VON GIMBORN GmbH) <br><br> * Revendication * <br><br> -- | 1,5 |
| | DE - A - 2 231 884 (H. VON GIMBORN GmbH) <br><br> * Exemple, revendication * <br><br> -- | 1 |
| | FR - A - 2 432 311 (P. FABRE S.A.) <br><br> * Exemples de shampooings; re-vendications * <br><br> -- | 1,2 |
| A | FR - A - 2 382 233 (OREAL) <br><br> * Page 8, ligne 38 - page 10, ligne 24; exemple 1; reven-dications * <br><br> -- | 7 |
| A | FR - A - 1 238 839 (MATTOSSIECH) <br><br> * Page 1, colonne de gauche, lignes 1-4, 16-24 le résumé * <br><br> -- | 3 |
| A | GB - A - 1 053 319 (C.A. HEWITT) <br><br> * Revendications * <br><br> -- | 4 |
| A | FR - A - 1 215 850 (LEVI VALENSIN) <br><br> * Résumé * <br><br> -- <br><br> ./. | 6 |

**DOCUMENTS CONSIDERES COMME PERTINENTS**

CLASSEMENT DE LA DEMANDE (Int Cl.³)

A 61 K 7/06
7/48

DOMAINES TECHNIQUES RECHERCHES (Int. Cl.³)

A 61 K 7/06
7/48
7/50

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cite dans la demande
L: document cite pour d'autres raisons

&: membre de la même famille. document correspondant

Le présent rapport de recherche a été établi pour toutes les revendications

| Lieu de la recherche | Date d'achèvement de la recherche | Examinateur |
|---|---|---|
| La Haye | 26-06-1981 | WILLEKENS |

OEB Form 1503.1   06.78

Office européen
des brevets

**RAPPORT DE RECHERCHE EUROPEENNE**

| DOCUMENTS CONSIDERES COMME PERTINENTS | | | CLASSEMENT DE LA DEMANDE (Int. Cl. ³) |
|---|---|---|---|
| Catégorie | Citation du document avec indication en cas de besoin, des parties pertinentes | Revendication concernée | |
| A | FR - A - 1 367 618 (CHOPPY)<br>* Page 1, colonne de gauche, lignes 1-3; page 2, tableau; le résumé * | 6 | |
| | -- | | |
| A | FR - A - 2 224 545 (BECHECLOUX DE LA BRECHE)<br>* Page 1, lignes 19-29; revendications * | 8 | |
| | ---- | | DOMAINES TECHNIQUES RECHERCHES (Int. Cl. ³) |

OEB Form 1503.2   06.78